# EUROPEAN PATENT SPECIFICATION

(11) **EP 0 422 883 B1**
(45) Date of publication and mention of the grant of the patent: **20.07.1994**
(21) Application number: 90311028.6
(22) Date of filing: 09.10.1990
(51) Int. Cl.: A23G 1/22, A23G 3/02, A23G 3/30, A23P 1/10, A61J 3/08

(54) **Starchless moulding process and apparatus**
Stärkefreies Formverfahren und Vorrichtung
Procédé de moulage sans amidon et appareil

(30) Priority: 10.10.1989 US 418597
(43) Date of publication of application: 17.04.1991
(73) Proprietor: WARNER-LAMBERT COMPANY, Morris Plains New Jersey 07950 (US)
(72) Inventor: Bunick, Frank John, Randolph, New Jersey 07869-5805 (US); Soliman, Ahmed Abd-El-Hamid, Budd Lake, New Jersey 07828 (US)
(74) Representative: Tesch, Rudolf, Dr.

(56) References cited:
- FR-A- 2 513 860
- GB-A- 2 190 093
- US-A- 3 981 656
- US-A- 4 739 963
- US-A- 4 759 937
- DATABASE WPIL no. 85-113911, 1985, Derwent Publications Ltd., GB; & JP - A - 60055950 (NIPPON CIBA-GEIGY) 01.04.1985

## Description

This invention relates to a starchless moulding process and apparatus, intended to replace the starch moulds used in the manufacture of food and non-food products.

Moulded products, such as confectionery products, are prepared by depositing a fluid mass into a preformed mould, allowing the fluid mass to solidify or gel therein, and removing the solidified or gelled product from the mould. Preformed depressions in a bed of dry powdered starch are the most commonly used technique for forming the mould.

There have been several recent developments in the moulding industry which have attempted to eliminate the use of starch, because starch processing has many drawbacks; for instance, dustiness is an obvious disadvantage and, associated with it, the explosion hazard. The continuous use of starch also creates a microbiological problem for, in spite of the required heating, drying and sifting, the starch is never sterile; in fact, moulding starch can have high bacterial counts. Sifting never eliminates all foreign matter and splinters of wood from broken trays and confectionery residues may contaminate the products. In addition, rodents can be a serious problem in the base of the moulding machines and can cause further serious contamination.

"Starchless" moulding technology has developed from the use of metal moulds with compressed air ejection of the pieces. In addition, rubber and silicone rubber moulds have been used for some types of fondant, caramels, and toffees. Plastic coated "drop" rolls have also been used for certain cremes and pastes. The starchless moulds are usually constructed in a tray form, each of which may contain a hundred or more depressions with the trays being operatively connected to one another to form a continuous belt or conveyer. In a continuous starchless moulding operation for soft confections, each mould is typically coated with a special release agent, the fluid confectionery recipe ingredients are then cast or deposited into the starchless mould (depositing), solidified therein (tempering), and mechanically ejected therefrom (demoulding), and then the moulds are cleaned and coated with fresh release agent for recycling. In demoulding, the mechanical ejection may be accomplished by mechanical fingers which force the gelled composition from a deformed flexible mould (e.g. moulds constructed of synthetic or natural rubber moulds) or by air expulsion from a rigid mould. One of the disadvantages with such starchless moulding techniques is the cost of preparing and changing moulds which is very high, resulting in the restricted use of such moulds to well established products with assured markets rather than to products being field tested on an experimental basis.

Starchless moulding procedures also require maintenance. In a continuous operation, each mould is cleaned so that it is free from solidified confectionery product and microbial contamination before recoating with fresh release agent and recycling. Critical processing factors need to be developed for effective air demoulding. These include the size and configuration of the holes within the base of the mould impression, the type of release agent and how it is applied, the mould design and construction, air pressure, cooling time and conditions, and so forth.

In this regard, incomplete release and non-uniform release of the solidified confectionery product from the mould are particularly troublesome problems since sugar solutions tend to stick to any mould surface. Numerous proposals have been made to improve upon the release of the solidified confectionery product from the mould. In an attempt to overcome these problems, polytetrafluoroethylene is conventionally used as a permanent mould coating. For soft confections, a temporary release agent coating (e.g. acetylated monoglyceride) is necessarily applied before each deposition of confection into the mould.

According to one aspect of the present invention, there is provided a process for making a moulded product, which comprises: depositing a molten or fluid mass of product ingredients into a porous non-starch hydrophilic polymeric mould; gelling or hardening the fluid mass while absorbing moisture or other solvent into the porous polymer by a wicking action; and recovering the formed product.

Preferably the process comprises:
a) preparing a fluid mass of product ingredients;
b) depositing the fluid mass into a solid mould comprising a porous non-starch hydrophilic polymeric plastics material;
c) removing moisture or other solvent from the deposited mass by absorption into the porous polymer by a wicking action;
d) gelling or solidifying the fluid mass within the mould to provide a moulded product; and
e) expelling the product from the mould.

Another aspect of the present invention provides a mould for forming a product, which comprises a mould shaped from a porous non-starch hydrophilic polymeric plastics material capable of wicking solvent or water from the product to be moulded.

As a result of the present invention, the disadvantages of starch moulding as well as those of "starchless moulding" techniques, have been obviated by the use of a porous non-starch hydrophilic polymer plastic which functions similar to starch.

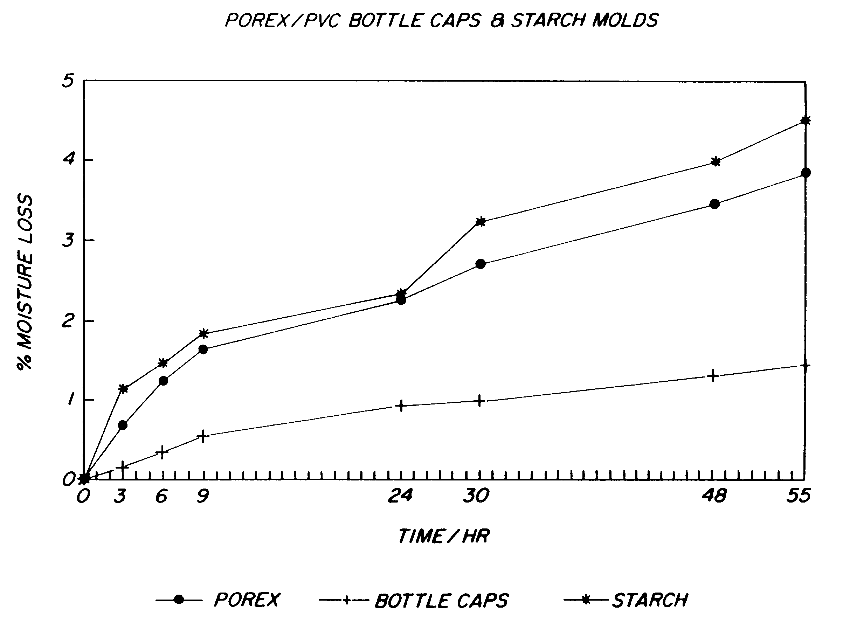

In the sole drawing referred to later, Figure 1 demonstrates moisture loss from various moulds as described in Example 1.

The use of the porous non-starch hydrophilic polymeric product allows starch to be replaced in traditional moulding operations. This applies not only to food items such as confections but also to non-food items such as pharmaceutical and cosmetic products. Thus the porous polymers have utility, when employed in the present invention, in the processing of a wide variety of product formulations currently being made by the starch casting technique. The application of porous polymers to wet casting eliminates the problems associated with starch casting such as explosion hazards, microbial contaminations, messy starch trays, starch dust, and so forth.

Porous polymers can be used when product formulations are deposited in a molten or fluid state, previously requiring starch for the removal of excess water or solvent. For moulding purposes, the porous polymers can be formed, cut or otherwise shaped into a variety of shapes and sizes. In confectionery applications, a large sheet of polymer would be formed into a mould containing many individual depressions into which a fluid mass is deposited.

Upon cooling, the polymer sheets containing deposited material would be stored under conditions required to complete drying of the product to an appropriate solids level. These conditions might range from ambient to controlled temperature and humidity conditions and may involve passing air or other gas over and/or below the porous polymer mould to facilitate removal of moisture or solvent from the "wick-like" porous polymer.

As with the techniques used with moulding starch, the porous polymer will be used to form the impressions for the deposited product. The impressions may be made on top of existing support frames or structures, such as those presently used in starch moulding. In addition to using existing supports, whole moulds may be prepared from the porous polymer itself provided suitable thickness are employed. Polymer thicknesses may range from 0.16cm (one-sixteenth inch) to several centimetres thick. Preferred structures are self supporting and have thicknesses from about 0.32cm to 1.27cm (one-eighth to about one-half inch) thick.

The products used to fill the mould should be in a molten or fluid state, that is either pourable into the mould or capable of being compressed by the mould. Such products usually contain from 5 to 85% by total weight of water or other solvent. Once placed in or compressed by the mould, the product will be left in the mould for a sufficient time to permit the water or solvent to "wick" into the porous polymer. This wicking action results in a drying effect causing the product to gel and/or harden. Suitable times will vary depending upon the product being made and the amount of water or other solvent that needs to be removed. Exemplary times may range from 15 minutes to several days and are preferably from at least 1 to 24 hours.

Unlike new starch which does not print well and requires the addition of mineral or vegetable oil to improve bonding, the porous polymers used in the present invention may be employed without such additives. The porous polymers form a mould which has a smooth surface resulting in products having a smooth surface, which can be easily removed from the mould.

It is also known that starch moulds need to be aerated. The mould board which prints the impressions compresses the starch in the tray. If the starch is too dense, it compacts badly and prevents deep mould impressions. Aeration is normally done after passing the starch through a fine sieve.

Unlike starch, the porous polymeric plastics material once compressed to form the mould need not be replaced following each moulding operation. This thus enables the moulds to be reused multiple times without concern about mould depth and cleaning. In addition, the porous polymers are capable of being aerated, like starch moulds, so that moisture or solvent picked up by the mould is easily removed. Such aeration procedures may entail passing air or other gas over and/or below the polymeric plastics material.

The use of the porous polymer plastics material in the formulation of the mould also eliminates the need to remove the tailings and fragments of deposited products that is required in starch moulding procedures. In addition, the present porous polymer moulds do not require heat to remove the moisture or solvent from the product as is required when using rubber or metal moulds which must be heated to high temperatures to form a solid product within the mould.

It should also be evident that the porous polymer moulding of the present invention avoids the dust, explosion hazards and microbial contaminations present with starch moulding operations. Furthermore, porous polymer moulding allows the use of traditional starch cast formulations where other starchless systems require formula modifications or special ingredients.

The porous polymers useful in the present invention comprise those polymers which are capable of wicking solvent or water from a product to be moulded. Exemplary polymers include synthetically produced plastic polymers and particularly those selected from high-density polyethylene, high molecular weight polyethylene, polypropylene, polypropylene, ethylene-vinyl acetate copolymers, polytetrafluoroethylene, styrene-acrylonitrile copolymers, polyvinylidene fluoride and mixtures thereof.

The porous polymers are hydrophilic in character and preferably have an average pore size of 0.8 to 2000 micrometres. Depending on the material to be moulded and the substance to be removed by wicking a specific polymer material may be selected to achieve maximum product hardening within the shortest time period. For example, when removing solvent from a cosmetic or pharmaceutical composition pore sizes of 35 to 500 micrometres have been found to be useful. Suitable material having this pore size may be obtained from high-density polyethylene (having an average pore size of from 35 to greater than 250 micrometres) and polypropylene (having an average pore size from 125 to 350 micrometres). In contrast, when removing water from a product such as confectionery product, pore sizes of from 5 to 100 micrometres have been found suitable. Such pore sizes can be obtained using high molecular weight polyethylene (having an average pore size of from 10 to 40 micrometres) and the above mentioned materials. Products having this pore size and porous structure may be obtained from Porex Technologies , U.S.A.

Any product which is capable of being prepared in a starch mould may be prepared in the moulds of the present invention. While not being limited to particular products a wide variety of confectionery, pharmaceutical and cosmetic products may be prepared using the porous polymer moulds of the present invention. Exemplary pharmaceutical and cosmetic products include tabletted and moulded products, stick deodorants, suppositories, lipstick and shaped cosmetic products.

The confectionery material may be selected from fondant creams, jellies, nut pastes, marzipans, turkish delights, soft caramels, fudges, marshmallows, gums and pastilles as well as other confectionery products that may be compressed and/or moulded such as shaker moulded products.

As indicated above, moisture or solvent may be removed by evaporation into the surrounding atmosphere or by passing air over and/or below the polymer mould to accelerate the procedure.

It should be appreciated that the mould may contain one or more port holes for air or steam injection to remove the product once formed. As in conventional operations, the present moulds may employ standard ejection procedures, such as the use of pressure being applied to the bottom of the mould to force the product out of the mould or compressed air or steam which passes through tiny holes in the bottom of each mould while they are tilted at an inverted angle, usually 45°. Critical processing factors for effecting pressure, air or steam demoulding are well within the skill of the ordinary skilled artisan and do not constitute a part of this invention.

In addition to the use of ejection means, the moulds of the present invention may be equipped with cooling means which circulate within the mould to aid in chilling the material to be hardened. Such means are well known for chocolate coating confectionery products.

The following example serves to provide further appreciation of the present invention. All percentages throughout the specification are by weight % of the final product unless otherwise indicated, wherein all percentages will total 100% of ingredients in the final composition.

### Example

This example compares the loss of moisture from a deposited product when added to various moulds.

A cooked gelatin solution (containing about 75% solids and about 25% water) was placed into three separate moulds having identical configurations. The amount of moisture loss was measured (by weight measurements) over a fifty five hour period and is recorded in Figure 1 of the accompanying drawing. The results indicate that moisture loss from mould A prepared from polyvinylchloride was significant lower than that achieved for moulds containing the inventive porous hydrophilic polyethylene (B) and a conventional starch mould (C).

## Claims

1. A process for making a moulded product, which comprises: depositing a molten or fluid mass of product ingredients into a porous non-starch hydrophilic polymeric mould; gelling or hardening the fluid mass while absorbing moisture or other solvent into the porous polymer by a wicking action; and recovering the formed product.

2. A process according to claim 1, which comprises:
a) preparing a fluid mass of product ingredients;
b) depositing the fluid mass into a solid mould comprising a porous non-starch hydrophilic polymeric plastics material;
c) removing moisture or other solvent from the deposited mass by absorption into the porous polymer by a wicking action;
d) gelling or solidifying the fluid mass within the mould to provide a moulded product; and
e) expelling the product from the mould.

3. A process according to claim 1 or 2, wherein the fluid mass of product contains from 5% to 85% of water.

4. A process according to claim 1 or 2, wherein the fluid mass of product contains from 5% to 85% of a solvent.

5. A process according to any preceding claim, wherein the product is a food or non-food item.

6. A process according to claim 5, wherein the product is a confectionery material.

7. A process according to claim 6, wherein the confectionery material is selected from fondant cremes, jellies, nut pastes, marzipans, turkish delights, soft caramels, fudges, marshmallows, gums and pastilles.

8. A process according to claim 5, wherein the product is a pharmaceutical product or a cosmetic product.

9. A process according to any preceding claim, wherein the porous polymer is selected from high-density polyethylene, high molecular weight polyethylene, polypropylene, ethylene-vinyl acetate copolymers, polytetrafluoroethylene, styrene-acrylonitrile copolymers, polyvinylidene fluoride, and mixtures thereof.

10. A process according to any preceding claim, wherein the porous polymer has an average pore size of from 0.8 to 200 micrometres.

11. A process according to any preceding claim, wherein air is passed over and/or under the porous polymer mould to facilitate removal of moisture or solvent from the porous polymer.

12. A process according to any preceding claim, wherein the gelled or hardened product has a consistency ranging from a pliable plastic mass to a pliable solidified mass.

13. A mould for forming a product, which comprises a mould shaped from a porous non-starch hydrophilic polymeric plastics material capable of wicking solvent or water from the product to be moulded.

14. A mould according to claim 13, wherein the mould is composed entirely of the porous non-starch hydrophilic polymeric plastics material capable of wicking solvent or water from the product to be moulded.

15. A mould according to claim 13, wherein the mould comprises an outer support structure and an inner layer containing the porous non-starch hydrophilic polymeric plastics material capable of wicking solvent or water from the product to be moulded.

16. A mould according to claim 13, 14 or 15, wherein the porous polymer capable of wicking solvent or water from the product to be moulded is selected from high-density polyethylene, high molecular weight polyethylene, polypropylene, ethylene-vinyl acetate copolymers, polytetrafluoroethylene, styrene-acrylonitrile copolymer, polyvinylidene fluoride, and mixtures thereof.

17. A mould according to any one of claims 13 to 16, wherein the porous polymer has an average pore size of from 0.8 to 2000 micrometres.

## Patentansprüche

1. Verfahren zur Herstellung eines Formlings durch Ablagern einer aufgeschmolzenen oder fließfähigen Masse von Produktbestandteilen in einer porösen, nicht aus Stärke bestehenden, hydrophilen polymeren Form, Gelieren oder Härten der fließfähigen Masse unter Absorption von Feuchtigkeit oder eines sonstigen Lösungsmittels in das poröse Polymer durch Dochtwirkung und Gewinnen des Formlings.

2. Verfahren nach Anspruch 1, umfassend (folgende Stufen):
a) Zubereiten einer fließfähigen Masse von Produktbestandteilen;
b) Ablagern der fließfähigen Masse in einer festen Form, umfassend ein poröses, nicht aus Stärke bestehendes, hydrophiles polymeres Kunststoffmaterial;
c) Entfernen von Feuchtigkeit oder eines sonstigen Lösungsmittels aus der abgelagerten Masse durch Absorption in das poröse Polymere infolge Dochtwirkung;
d) Gelieren oder Verfestigen der fließfähigen Masse innerhalb der Form zur Bildung eines Formlings und
e) Austreiben des Produkts aus der Form.

3. Verfahren nach Anspruch 1 oder 2, wobei die fließfähige Produktmasse 5 - 85% Wasser enthält.

4. Verfahren nach Anspruch 1 oder 2, wobei die fließfähige Produktmasse 5 - 85% eines Lösungsmittels enthält.

5. Verfahren nach einem der vorhergehenden Ansprüche, wobei es sich bei dem Produkt um ein Nahrungsmittel oder Nicht-Nahrungsmittel handelt.

6. Verfahren nach Anspruch 5, wobei es sich bei dem Produkt um eine Süßware bzw. ein Zuckerwerk handelt.

7. Verfahren nach Anspruch 6, wobei das Zuckerwerk aus Fondantcremes, Gallerten bzw. Fruchtgelees, Nußpasten, Marzipansorten, Geleefrüchten, feinem Konfekt, Fondant, Weichkaramellen bzw. weichem Zuckerwerk, Marshmallows, Gummies und Pastillen ausgewählt ist.

8. Verfahren nach Anspruch 5, wobei das Produkt aus einem pharmazeutischen Produkt oder einem kosmetischen Produkt besteht.

9. Verfahren nach einem der vorhergehenden Ansprüche, wobei das poröse Polymer aus hochdichtem Polyethylen, hochmolekularem Polyethylen, Polypropylen, Ethylen/Vinylacetat-Copolymeren, Polytetrafluorethylen, Styrol/Acrylnitril-Copolymeren, Polyvinylidenfluorid und Mischungen derselben ausgewählt ist.

10. Verfahren nach einem der vorhergehenden Ansprüche, wobei das poröse Polymer eine durchschnittliche Porengröße von 0,8 - 200 »m aufweist.

11. Verfahren nach einem der vorhergehenden Ansprüche, wobei Luft zur leichteren Entfernung von Feuchtigkeit oder Lösungsmittel aus dem porösen Polymer über und/oder unter die poröse Polymerform geleitet wird.

12. Verfahren nach einem der vorhergehenden Ansprüche, wobei das gelierte oder gehärtete Produkt eine Konsistenz von einer geschmeidigen plastischen Masse bis zu einer geschmeidigen verfestigten Masse aufweist.

13. Form zur Ausformung eines Produkts, die aus einem porösen, nicht aus Stärke bestehenden, hydrophilen polymeren Kunststoffmaterial mit der Fähigkeit zum Aufsaugen (durch Dochtwirkung) des Lösungsmittels oder von Wasser aus dem zu formenden Produkt hergestellt ist.

14. Form nach Anspruch 13, die vollständig aus dem porösen, nicht aus Stärke bestehenden hydrophilen polymeren Kunststoffmaterial mit der Fähigkeit zum Aufsaugen (infolge Dochtwirkung) eines Lösungsmittels oder von Wasser aus dem auszuformenden Produkt besteht.

15. Form nach Anspruch 13, umfassend eine äußere Tragestruktur und eine innere Schicht mit dem porösen, nicht aus Stärke bestehenden, hydrophilen polymeren Kunststoffmaterial mit der Fähigkeit zum Aufsaugen (infolge Dochtwirkung) eines Lösungsmittels oder von Wasser aus dem auszuformenden Produkt.

16. Form nach Anspruch 13, 14 oder 15, wobei das poröse Polymer mit der Fähigkeit zum Aufsaugen (infolge Dichtwirkung) eines Lösungsmittels oder von Wasser aus dem auszuformenden Produkt aus hochdichtem Polyethylen, hochmolekularem Polyethylen, Polypropylen, Ethylen/Vinylacetat-Copolymeren, Polytetrafluorethylen, Styrol/Acrylnitril-Copolymeren, Polyvinylidenfluorid und Mischungen derselben ausgewählt ist.

17. Form nach einem der Ansprüche 13 bis 16, wobei das poröse Polymer eine durchschnittliche Porengröße von 0,8 - 2000 »m aufweist.

## Revendications

1. Un procédé de préparation d'un produit moulé, qui comprend:
- le dépôt d'une masse à l'état fondu ou fluide des ingrédients du produit dans un moule comprenant un polymère hydrophile poreux différent de l'amidon;
- la gélification ou le durcissement de la masse fluide tout en absorbant l'humidité ou un autre solvant dans le polymère poreux par capillarité ou action de mèche; et
- la récupération du produit formé.

2. Un procédé selon la revendication 1, qui comprend:
a) la préparation d'une masse fluide des ingrédients du produit;
b) le dépôt de la masse fluide dans le moule solide comprenant un matériau plastique polymère hydrophile poreux différent de l'amidon;
c) l'elimination de l'humidité ou d'un autre solvant de la masse déposée par absorption dans le polymère poreux par capillarité ou action de mèche;
d) gélification ou solidification de la masse fluide dans le moule pour fournir un produit moulé; et
e) éjection du produit du moule.

3. Un procédé selon la revendication 1 ou 2, dans lequel la masse fluide du produit contient de 5 à 85% d'eau.

4. Un procédé selon la revendication 1 ou 2, dans lequel la masse fluide du produit contient de 5 à 85% d'un solvant.

5. Un procédé selon l'une quelconque des revendications précédentes, dans lequel le produit est un article alimentaire ou un article non alimentaire.

6. Un procédé selon la revendication 5, dans lequel le produit est un produit de confiserie.

7. Un procédé selon la revendication 6, dans lequel le produit de confiserie est choisi dans le groupe consistant en crèmes fondantes, gelées, pâtes de noix, massepains, Rahat loukoums, caramels mous, fondants américains, guimauves, gommes et pastilles.

8. Un procédé selon la revendication 5, dans lequel le produit est un produit pharmaceutique où un produit cosmétique.

9. Un procédé selon l'une quelconque des revendications précédentes, dans lequel le polymère poreux est choisi parmi le polyéthylène haute densité, le polyéthylène de haut poids moléculaire, le polypropylène, les copolymères éthylène-acétate de vinyle, le polytétrafluoroéthylène, les copolymères styrène-acrylonitrile, le poly(fluorure de vinylidène), et leurs mélanges.

10. Un procédé selon l'une quelconque des revendications précédentes, dans lequel le polymére poreux présentent une dimension de pores moyenne de 0,8 à 200 micromères.

11. Un procédé selon l'une quelconque des revendications précédentes, dans lequel on fait passer de l'air sur et/ou sous le moule en polymère poreux pour faciliter l'élimination de l'humidité ou du solvant du polymère poreux.

12. Un procédé selon l'une quelconque des revendications précédentes, dans lequel le produit gélifié ou durci présente une consistance allant de la masse plastique pliable vers une masse solidifiée pliable.

13. Un moule pour former un produit, qui comprend un moule formé à partir d'un matériau plastique polymère hydrophile poreux différent de l'amidon, susceptible d'absorber, par capillarité ou effet de mèche, de l'eau ou un solvant à partir du produit devant être moulé.

14. Un moule selon la revendication 13, dans lequel le moule est composé entièrement du matériau plastique polymère hydrophile poreux différent de l'amidon capable d'absorber, par capillarité ou effet de mèche, l'eau ou un solvant à partir du produit devant être moulé.

15. Un moule selon la revendication 13, dans lequel le moule comprend une structure extérieure de support et une couche interne contenant le matériau plastique polymère hydrophile poreux différent de l'amidon susceptible d'absorber, par capillarité ou effet de mèche, le solvant ou l'eau à partir du produit devant être moulé.

16. Un moule selon l'une des revendications 13, 14 ou 15, dans lequel le polymère poreux susceptible d'absorber par capillarité ou effet de mèche du solvant ou de l'eau à partir du produit devant être moulé est choisi dans le groupe consistant en polyéthylène haute densité, polyéthylène de haut poids moléculaire, polypropylène, copolymère éthylène-acétate de vinyle, polytétrafluoroéthylène, copolymères styrèneacrylonitrile, poly(fluorure de vinylidène), et leurs mélanges.

17. Un moule selon l'une quelconque des revendications 13 à 16, dans lequel le polymère poreux présente une dimension de pores moyenne de 0,8 à 2 000 micromètres.
